# EUROPEAN PATENT APPLICATION

(11) **EP 1 514 877 A1**
(43) Date of publication of application: **16.03.2005**
(21) Application number: 03729798.3
(22) Date of filing: 09.05.2003
(51) Int. Cl.: C08B 37/16, A61K 47/40

(54) **COMPLEX OF ORGANIC MEDICINES AND BETA-CYCLODEXTRIN DERIVATIVES AND ITS PREPARING PROCESS**

(30) Priority: 10.05.2002 CN 02116766
(71) Applicant: Liu, Yunqing, Haidan District, Beijing 100083 (CN); Liu, Xiying, Haidan District, Beijing 100083 (CN); Liu, Wei, Haidan District, Beijing 100083 (CN); Liu, Tong, Haidan District, Beijing 100083 (CN)
(72) Inventor: Liu, Yunqing, Haidan District, Beijing 100083 (CN); Liu, Xiying, Haidan District, Beijing 100083 (CN); Liu, Wei, Haidan District, Beijing 100083 (CN); Liu, Tong, Haidan District, Beijing 100083 (CN)
(74) Representative: Goddar, Heinz J., Dr.
(86) International application number: PCT/CN2003/000337
(87) International publication number: WO 2003/095498

(57) **Abstract**

The present invention relates to a process of preparing a water-soluble complex of water-insoluble or sparingly-soluble organic medicines and beta-cyclodextrin derivatives: a. dissolving successively or simultaneously the organic medicines and the beta-cyclodextrin derivatives in organic solvent(s) at certain mole ratio or mass ratio in the presence of suitable amount of water; b. removing the organic solvent from the solution of the step a to obtain aqueous solution of the organic medicine and beta-cyclodextrin derivative; and c. removing water from the aqueous solution to obtain the complex of the organic medicine and beta-cyclodextrin derivative. The invention also provides a sterile water-soluble complex of water-insoluble or sparingly-soluble organic medicines and beta-cyclodextrin derivatives. A fully-water-soluble complex can be prepared from any water-insoluble or sparingly-soluble organic medicines or other organic compounds according to the present invention. Thus, appreciably convenient means are provided for industrial uses of such organic medicine preparations and of other organic compounds.

## Description

### Field of the Invention

The present invention relates to a complex of organic medicines and beta-cyclodextrin derivative and methods for preparing the complex and methods for using the complex.

### Background of the Invention

As the solubility of medicines in water is below certain order of magnitude, the development and bioavailability of its preparation is restricted and influenced. However, water-insoluble or sparingly-soluble organic medicines account for about one-third or more in pharmacopeia, collected edition of general medicines and among active medicines screened with chemical compounding methods. It is important that solubility of sparingly-soluble or water-insoluble medicines in water is increased to improve the efficacy of such medicines.

The method of increasing water-solubility involves complexing between beta-cyclodextrin (β-CD) or its derivatives and the medicine has been studied widely for many years. But the low-solubility of beta-cyclodextrin restricts its applications, especially via intravenous injection, as β-CD can increase the amount of blood urea nitrogen (BUN). While it is absorbed in renal tubules, β-CD is present in crystal form due to its low-solubility, resulting in tissues necrosis.

In recent years, hydroxy propyl-beta-cyclodextrin (HP-β-CD) as β-CD derivatives has been used to prepare all kinds of dose types for intravenous injection and oral administration, which is characterized by large safe dose, nice solubility with blood, steady medicine efficacy, increasing water-solubility and stability, therefore, it is believed as a promising medicine carrier material.

That beta-cyclodextrin derivative as medicines to carrier prepare inclusion complex can increase medicines solubility is found in many reports and patents. Therein, Pitha et. al.( Int J Pharm 1986.29(1):73) reports 32 medicines were solubilized with concentrated solution of hydroxy propyl-beta-cyclodextrin (2-HP-β-CD) (40∼50%), resulting in solubility of these compounds increased by 1.3-13666 times. Plenty of similar preparations were also introduced in No.4,727,064 of US patent, proving that the water solubility of the inclusion complex of beta-cyclodextrin (β-CD) derivatives and medicines is well. Most of the methods of preparation are solution equilibration methods, in which the deposition is discarded and part of solution is used as medicines by means of solubilization effect of concentrated solution of hydroxy propyl-beta-cyclodextrin. It is also processed to solid powder by means of crystallization and lyophilization, then the insoluble substance may appear after the powder is dissolved in water. Furthermore, quite a few overview literatures exist, for instance, Zhang xiurong, et. al. (China pharmacy Journal 2000.38 (10): 649) introduced beta-cyclodextrin derivatives applied in non-intestinal tract administration; Xie botai, et al. (overseas medicine - fascicule of synthesized medicines, biochemical medicines, preparation 2002.23 (5): 302) introduced application of hydroxy propyl-beta-cyclodextrin in pharmacy, etc., these all increased the solubility in water as medicines carrier.

Artemisinin is an excellent antimalaria agent invented by Chinese scientists, but its solubility is poor. The fact that its solubility was increased by synthesis derivative took little effect. It is convenient to increase solubility with cyclodextrin as carrier. WO90/02141 contains many preparation methods of compounds. In it, for instance, Artemisinin derivative is dissolved in cyclodextrin derivatives solution, or in organic solvent then mixing the cyclodextrin derivatives aqueous solution with stirring, and crystal separated out, etc. Switzerland monopoly 685391 showed that, Artemisinin and its derivatives and cyclodextrin derivatives were mixed and suspended in water simultaneously, then the mixture was stirred for more than 30 hours in 37°Cwater bath, and insoluble substance removed by filtration, and the solution split charged in xilin bottle, freeze-dried. Each bottle of Artemisinin contains about 50mg. A similar method was used in more than 10 practice examples of the literature. Up to now, solubilization with beta-cyclodextrin as medicines carrier is mostly used to non-intestinal tract administration and intravenous drip, but is not used widely to oral administration, which causes may be the facts that simple methods of industrialization manufacturing have not been yet discovered.

Summarizing preparation methods of the literatures of many recent years: 1. based on solublization of critical micelle concentration, with HP-β-CD aqueous solution of superior concentration (e.g.>40%), dissolve sparingly-soluble medicines; 2. by means of mechanical mix and ultrasonic, disperse the solid medicines highly to promote its solution; 3. by means of feasible acid and alkali surface active agent, cosolvent, increase solublization and its stability. 4. separate out precipitation from saturated solution by means of crystallization and coprecipitation method, then spray dry or freeze dry it; 5. due to long technics cycle, deal with the medicines in clean circumstance and low temperature for several decades, hours or several days. Itraconazole injection was intravenous injection liquid preparation for the inclusion complex of Itraconazole-hydroxide propyl beta-cyclodextrin, and the first medicine admitted into clinical trial in 2000. Quite an amount of acid and cosolvent was applied in its compounding formula, in order to increase stability of preparation. Mass ratio of medicine and hydroxide propyl beta-cyclodextrin is 1:40. In the report of "a study on preparation and features for the solid inclusion complex of norfloxacin-hydroxide propyl beta-cyclodextrin"in 17(3), 2000, 6, Spectroscopy Journal, with HP-β-CD aqueous solution of superior concentration, by mechanical agitation, in clean circumstance and low temperature for several decades, hours or several days, and by means of coprecipitation method, and by drying, obtain solid powdered the inclusion complex of norfloxacin-hydroxide propyl beta-cyclodextrin.

The inclusion complex prepared with the method increases medicine dissolubility to some extent, but the solid powdered inclusion complex may not be always dissolved completely when added into water, or the solution is diluted when added into water again to separate out insoluble substances. Therefore, its characteristic is instability and irreversibility to dilution and redissolving, for its component instability, since the fact that the solid powder is an inclusion complex, and not a stable complex compound formed of Supramolecular chemistry.

### Summary of the Invention

The objective of the invention lies in providing a preparation method of complex of organic medicine and beta-cyclodextrin derivatives and complex with this preparation method. The complex with this preparation method is component stable, and is a complex compound in the field of Supramolecular chemistry, which is extremely freely or freely soluble in water, with rapid dissolving speed and infinite dilution stability.

The invention provides a preparation method of complex of organic medicine and beta-cyclodextrin derivatives, which includes:
A. Dissolving successively or simultaneously the organic medicines and the beta-cyclodextrin derivatives in organic solvent(s) at certain mole ratio or mass ratio in the presence of a suitable amount of water;
B. Removing the organic solvent(s) from the solution of act A to obtain an aqueous solution of the organic medicines and beta-cyclodextrin derivatives; and
C. Removing water from the aqueous solution to obtain the complex of the organic medicines and beta-cyclodextrin derivatives.

In a preferred example, the organic solvent(s) is hydrophilic.

In another preferred example, the hydrophilic organic solvent(s) is selected from ester solvent of lower fatty acid, hydrocarbon solvent, halogenated hydrocarbon solvent, furan solvent, amide solvent, lower fatty alcohol, nitrile solvent, ketone solvent, and mixtures thereof.

In another preferred example, the hydrophilic organic solvent(s) is selected from methyl acetate, ethyl acetate, butyl acetate, petroleum ether, cyclohexane, methylene chloride, chloroform, water, tetrahydrofuran, dimethylacetamide, dimethyl formamide, methanol, ethanol, propanol, butanol, acetonitrile, acetone, and mixtures thereof.

In another preferred example, dissolving of act A is carried out at 30∼100°C.

In another preferred example, dissolving of act A is carried out at 60∼75°C.

In another preferred example, mole ratio or mass ratio of the organic medicines and the beta-cyclodextrin derivatives is critical mole ratio or critical mass ratio, or less than critical mole ratio or critical mass ratio, or more than critical mole ratio or critical mass ratio.

In another preferred example, critical value of the organic medicines and the beta-cyclodextrin derivatives is determined by means of unit test or preparing saturated solution.

In another preferred example, the beta-cyclodextrin derivative is selected from hydroxide propyl beta-cyclodextrin, hydroxyethyl beta-cyclodextrin, sulphoalkyl beta-cyclodextrin, ether beta-cyclodextrin, methyl beta-cyclodextrin, and ethyl beta-cyclodextrin.

In another preferred example, the beta-cyclodextrin derivative is hydroxide propyl beta-cyclodextrin.

In another preferred example, the method also comprises the act of sterile filtration of solution obtained in act A.

In another preferred example, the organic medicines are water-insoluble or sparingly-soluble organic medicines.

In another preferred example, the organic medicines are selected from at least one medicine indicated in table 1 and random their mixture.

In another preferred example, the methods comprise that the complex is heated and expanded in vacuum, dried, and made into multihole sterile granula or powder.

In another preferred example, the methods comprise that the complex is directly sub-packed and prepare power or freeze drying powder, mass or solution for injection.

In another preferred example, the methods comprise that the complex is used together with adjuvant approved in pharmacy to prepare tablet, granula, capsule, pill, chewing agent, suppository, or adhibition agent.

In another preferred example, the methods comprise that the complex is made into aerosol inhalation.

In another preferred example, the methods comprise that the complex is made into pharmaceutical solution for eye drip, nasal drip, gargle, inhalator, rectum, or wash.

In another preferred example, the methods comprise that the complex is made into medicines of prevention and treatment of pathogenic insects, hormone and nutrient additive, which are applied to domestic animals, poultries, agronomic crops and plants except humans.

In another preferred example, the methods comprise that the complex is used to prepare enzyme preparation and catalyst in chemical industry.

The present invention also provides a complex of the organic medicines and beta-cyclodextrin derivatives prepared with any the method.

The present invention also further provides a complex containing water-insoluble or sparingly-soluble organic medicines and beta-cyclodextrin derivatives.

In a preferred example, the organic medicines are selected from at least one indicated in table 1.

In another preferred example, mole ratio or mass ratio of the beta-cyclodextrin derivatives and the organic medicines existing in the complex, is critical mole ratio or critical mass ratio.

In another preferred example, mole ratio or mass ratio of the beta-cyclodextrin derivatives and the organic medicines existing in the complex, is more than critical mole ratio or critical mass ratio.

In another preferred example, mole ratio or mass ratio of the beta-cyclodextrin derivatives and the organic medicines existing in the complex, is less than critical mole ratio or critical mass ratio.

In another preferred example, the complex also contains an excipient, carrier or diluent agent approved in pharmacy.

### Description of the of the invention

The method of the present invention is the fact that according to principles of intermolecular interactions, as a subject of beta-cyclodextrin derivatives and an object of molecule of organic medicine or other organic molecule, in the necessary condition that water is present, a stable coordination compound (or named as complex, supramolecular compound) bonded with weak link (non-link) is formed, and a stable and freely soluble complex is prepared from organic medicine and beta-cyclodextrin derivatives.

The craft cycle of the invention is brief, and 3-4 hours is generally needed, comprising the procedures below:
- A.: In close reactor under clean surroundings, dissolve organic medicines in hydrophilic organic solvent, at the same time heat it at 30∼100°C until dissolved if necessary. Mix up solution of organic medicine and solution of beta-cyclodextrin derivatives of critical mole ratio or critical mass ratio;
- B.: Heat mixed solution obtained in act A, and remove the solvent, obtain an aqueous system of medicine and beta-cyclodextrin derivatives,
- C.: Decompress and concentrate the solution obtained in act B, make its water content to reach to normal content, hence obtain an expanding loose body as complex of medicine and beta-cyclodextrin derivatives.

In accordance with the demands of pharmaceutics, the complex in form of expanding loose body prepared with the method of present invention can be used to prepare below preparations:

The powder for injection can be prepared directly by subpackaging; the solution or freeze-dried powder or mass can be prepared by conversion of dissolving; all kinds of oral and cavum administrations such as tablet, granula, capsule, syrup, can be prepared after adding relevant adjuvants; powder spray inhalation is prepared; pharmaceutical solution for eye drip, nasal drip, gargle, inhalator, rectum, or wash is prepared; medicines of prevention and treatment of pathogenic insects, hormone and nutrient additive, which are applied to domestic animals, poultries, agronomic crops and plants except humans, can be prepared; it can be used for enzyme preparation and catalyst in chemical industry.

In act A, medicine and beta-cyclodextrin can also be dissolved simultaneously in suitable solvent according to critical mole ratio, to obtain mixed solution. In addition, according to the demands of preparation, the solution can be sterilized by ultrafiltration, decolorization and pyrogen is eliminated. In the course of solution, heating may be used according to variation of medicine if necessary in order to promote dissolving. The temperature of heating is commonly 30∼100 °C.

The temperature of decompression and concentration in act C is commonly 30∼100°C, optimum 60∼75°C. In this act, in reactor dry loose body is crashed into multihole particles or powder required in particle size. Water content of the medicine after drying can be determined aptly on basis of difference of medicine and preparation. Water content is optimized below 1wt%, in order to be convenient to process expanding loose body into particles or powders, etc.

The critical mole ratio or critical mass ratio in the present invention refers to the mole ratio and mass ratio when complex of organic medicine and beta-cyclodextrin derivatives prepared with the method of this invention is dissolved exactly in water of specified amount to form stable aqueous solution.

In the method of the present invention, in order to dissolve the organic medicine completely in water to keep stable, the critical mole ratio or critical mass ratio is preferentially selected in compounding ratio of organic medicine and beta-cyclodextrin derivatives. However, when certain medicine preparations need not be dissolved completely, compounding ratio of the medicine and beta-cyclodextrin derivatives may be less than critical mole ratio or critical mass ratio. In addition, if necessary, compounding ratio of the medicine and beta-cyclodextrin derivatives may be more than critical mole ratio or critical mass ratio. But in consideration of the factors such as cost of beta-cyclodextrin derivatives, excessive beta-cyclodextrin derivativ is generally unnecessary.

The critical value of organic medicine and beta-cyclodextrin derivatives in the present invention, may be determined with the undermentioned unit test method.

All kinds of mole ratio and mass ratio of the medicine and beta-cyclodextrin derivative is made into complex with expanding loose body according to the method of the present invention, then the set water is added into the a variety of compounding ratio of complex, and by observing properties of the solution, the compounding ratio of exactly dissolving in water and with stable property, acts as the critical mole ratio or critical mass ratio of the present invention.

With the method, critical value of complex of Artemisinin (molecular weight: 282.34, hardly water-soluble) and hydroxide propyl beta-cyclodextrin (mean molecular weight is about 1600) is measured.

| Mole ratio | mass ratio | product property |
|---|---|---|
| 3 | 16.98 | partly water-soluble |
| 4 | 22.64 | water-soluble, instable |
| *5 | 28.30 | freely soluble, stable |
| 6 | 33.94 | freely water-soluble, stable |

The above results indicate that a water-soluble complex can be generated as mole ratio of Artemisinin and hydroxide propyl beta-cyclodextrin is equal to or more than 1:5. Thereby, critical mole ration of complex of Artemisinin and hydroxide propyl beta-cyclodextrin is about 5, and mass ratio about 28.

The critical mole ratio and critical mass ratio in the present invention may be determined with the undermentioned saturated solution method.

Weighing accurately medicine, and placing in the container, and gradually adding beta-cyclodextrin aqueous solution metered accurately with agitation, until the medicine is dissolved exactly and completely in this aqueous solution. Calculate both mass number here and convert it to mole number. Repeat the above-mentioned test, and get the mean mole number of repeated test, and calculate critical mole ratio with the undermentioned formula:

Critical mole ratio of medicine=mean mole number of beta-cyclodextrin/ mean mole number of medicine

For example, critical mole ratio of Artemisinin and hydroxide propyl beta-cyclodextrin is measured with the undermentioned method: Weight accurately Artemisinin, and grind in tissue grinding device, and place in beaker, and gradually add 40% hydroxide propyl beta-cyclodextrin aqueous solution metered accurately while agitation, until Artemisinin is dissolved exactly and completely in this aqueous solution. Calculate both mass number here and convert it to mole number. Repeat the above-mentioned test 3 times, and get the mean mole number of repeated test, and calculate critical mole ratio of Artemisinin with the above-mentioned formula: it is equal to about 5.

From the results, it is seen that the results two measuring method of critical value are consistent generally. The critical mole ratio orcritical mass ratio of all kinds of medicines applied in the present invention, which measured in the experiments, is indicated in the following table 1:

**Table 1**

| medicine name | | | Critical value | |
|---|---|---|---|---|
| | | | mole ratio | Mass ratio |
| A. | antimalarial: sesquiterpene lactone Artemisinin and its derivatives | Artemisinin | 5 | |
| | | Dihydroartemisinin | 7 | |
| | | Artemether | 7 | |
| | | Artesunate | 3 | |
| | | Artemoter | 8 | |
| B. | macrolide antibiotics | Erythromycin | 6 | |
| | | Medicamycin | 8 | |
| | | Roxithromycin | 18 | |
| | | Azithromycin | 16 | |
| | | Clarithromycin | 18 | |
| C. | azole antifungal | Itraconazole | 18 | |
| | | Ketoconazole | 3 | |
| | | Miconazole | 6 | |
| | | Clotrimazole | 4 | |
| D. | alkaloid analgesic | Lappaconitine | 2 | |
| | | Rotundine | 1 | |
| E. | anxiolytic, sedative, hypnotic | Diazepam | 3 | |
| | | Lorazepam | 3 | |
| | | Estazolam | | 20 |
| | | Oryzanol | | 38 |
| | | Zolpidem | 2 | |
| F. | antineoplastics | Tamoxifen | 2 | |
| | | Taxol | 100 | |
| | | Busulfan | 5 | |
| | | Etoposide | 10 | |
| G. | steroid | prednisone | 4 | |
| | | testosterone | 4 | |
| | | Prasterone | 3 | |
| | | spironolactone | 3 | |
| H. | immunosuppressant | Ciclosporin | 66 | |
| I. | anlibechic, expectorant | Dioxopromethazine | | 2 |
| | | Bromhexine | 3 | |
| J. | Sulfa and synergist | sulfamethoxazole ,SMZ | 2 | |
| | | sulfafurazole | 2 | |
| | | sulfadiazine, SD | 3 | |
| | | TMP | 3 | |
| K. | antibacterial agent | Ampicilline | 3 | |
| | | Ciprofloxacin | 5 | |
| | | furazolidone | | 25 |
| | | norfloxacin | | 30 |
| | | Metronidazole | | 9 |
| | | Tinidazole | | 6.5 |
| L. | Antipyretic, anti-inflammatory agent, analgesic | paracetamol | | 3 |
| | | Piroxicam | | 15 |
| | | Ibuprofen | | 8 |
| M. | medicines for circulatory system | nimodipine | 35 | |
| | | nitrendipine | 3 | |
| | | cinnarizine | 3 | |
| | | molsidomine | | 11 |
| | | Lovastatin | 26 | |
| | | simvastatin | 29 | |
| N. | other and volatile material | famotidine | | 9 |
| | | Sibutramine | | 2 |
| | | Sildenafil | 2 | |
| | | gliclazide | 2 | |
| | | difenidol | 1 | |
| | | caffein | | 4 |
| | | ketotifen | | 4 |
| | | lipoic acid | 1 | |
| | | thioctamide | | 7 |
| | | menthol | 2 | |
| | | borneol | | 6 |
| | | camphor | | 8 |
| | | ligustrazine | | 12 |
| | | chlorbutanol | | 6 |
| | | vanillin | | 9 |

In the present invention, using mole ratio or mass ratio is based on property of organic medicine and convenience of calculation, and there is no substantial differece between the mole ratio and mass ratio

In the present invention, beta-cyclodextrin derivatives generally used are selected from hydroxide propyl beta-cyclodextrin, hydroxyethyl beta-cyclodextrin, sulphoalkyl beta-cyclodextrin, ether beta-cyclodextrin, and methyl beta-cyclodextrin, and ethyl beta-cyclodextrin hydroxide propyl beta-cyclodextrin is optimum.

The molecular formula of hydroxide propyl beta-cyclodextrin is (C₆H₁₀O₅)₇ • (C₃H₆O) n, MS≈5, and its molecular weight range from 1367∼1716, 1600 of mean molecular weight, containing 22.0∼41.0% of hydroxide oxypropyl group (-C₃H₆O) if calculated with dried products.

The solvent used in the present invention may be anyone of hydrophilic organic solvent. On basis of the demand of pharmaceutics, a suitable solvent should meet the following demand generally: it has appropriate solubility for medicines and vectors, is easily removed the minimum-remaining solvent and has no influence to the safety of the medicine and is soluble in water and removed easily by decompression.

The selected solvents in the present invention comprise: esters: lower fatty acid, such as methyl acetate, ethyl acetate, butyl acetate, etc.; hydrocarbon solvent: petroleum ether, cyclohexane, etc.; halogenated hydrocarbon: methylene chloride, chloroform; water, furan solvent; amide solvent: dimethylacetamide, dimethyl formamide, etc.; alcohols: low-grade fatty alcohol; nitrile: acetonitrile, ketone solvent: acetone, etc. These solvents can not only be used singly, but also with mixtures of two or more than two solvents.

"In the presence of suitable amount of water" in method of the present invention is necessary. But amount of water is not specially defined. The suitable water may be water added in, also water existing in organic medicine or beta-cyclodextrin derivatives. As the water exists in organic medicine or beta-cyclodextrin derivatives, additional water may not be added in the solvent.

The method of the present invention is applicable to any insoluble or sparingly-soluble organic medicine. The invention is illustrated with the undermentioned methods and examples conducted. It is notable that these methods and examples conducted are only used to better understand and conduct the invention, not to restrict the invention.

### Preferred Embodiments of the Invention

The complex of medicines and beta-cyclodextrin derivatives in the present invention prepared, comprises commonly the undermentioned factors:
1. Solvent: the appropriate solvent is selected on basis of physical- chemical property of medicine and the demand of solvent.
2. Compounding: medicine and carrier (beta-cyclodextrin derivatives) are compounded with the ratio determined in the experiment (critical mole ratio or critical mass ratio).
3. Dissolving and processing of solution: it is heated and color and pyrogen is removedif necessary. The method according to the solution after dissolved, and filtration treatment made, and settled solution obtained (the process of oral preparation may be simplified).
4. Concentration and conversion: filter liquor is input to rotation pot, and decompressed, concentrated in 120r/m of rotation rate, in the presence of heating, and the solvent is exactly removed, converted into aqueous system, finally materiel indicates vitroclastic. Homogeneous system is necessarily maintained during concentration and conversion, without insoluble substance separating out.
5. Expanding and drying: expanding is conducted at 120r/m of rotation rate, while drying at slow speed of rotation. The materiel of act 4 is heated continuously, and decompressed, dehydrated and expanded, and appears white or opaque, and foamed, uplifted, until dried, and finally multihole solid is obtained.
6. Crashing and discharging: the loose multihole materiel expanded is crashed into particles or powder according to the demand thendischarged.
7. Devices: rotation pot (with volume of 1,5,10,20,50 litres or more ) controlable temperature(0∼100°C), rotation rate: 0∼150r/min, adjustable, attached with solvent condensation and retrieving arrangement. vacuum pump: 30∼80 litres/min water flushing pump(or other pumps), vacuum degree: 0.1Mpa.

Property marker of complex of Medicine- beta-cyclodextrin derivatives: freely water-soluble, dilution stability with not less than 8 hours, in accordance with the demand of intravenous injection except oral administration.

### Example 1

Process of preparing a complex of Artemisinin-hydroxide propyl beta-cyclodextrin for injection

2g Artemisinin and 56g hydroxide propyl beta-cyclodextrin (critical mole ratio of the two is 5) are dissolved in appropriate amount of ethanol by heating. After 1g active carbon is added, the solution is boiled slightly for 15 minutes, and cooled down to about 50°C, and filtered. The filter liquor is added in rotation pot, and kept at 70∼80°C. The liquor is decompressed and concentrated before solvent is recycled and converted to aqueous system, then continuously decompressed until the materiel is expanded and dried for 2.5∼3h. The total time is about 3.5∼4h. yield: ≥98%(vary as batch charge). Solvent recovery rate: 85∼90%.

### Example 2

### Process of preparing a complex of Artesunate-hydroxide propyl beta-cyclodextrin for injection

10g Artesunate and 42g hydroxide propyl beta-cyclodextrin (critical mole ratio of the two is 3) are dissolved in ethanol. After slight amount of active carbon is added, the solution is heated for 15 minutes while agitation, and filtered. The filter liquor is added in rotation pot with volume of 1 litre, and kept at 70∼80°C in 120r/min rotation speed. The liquor is decompressed and concentrated before solvent is recycled and converted to aqueous system, then continuously decompressed until the materiel is expanded and dried for 2.5∼3h. The total time is about 3.5∼4h. yield: ≥98%. Solvent recovery rate: 85∼90%.

Complex of other Artemisininin are similar to the above-mentioned.

### Example 3

### Process of complex of Erythromycin-hydroxide propyl beta-cyclodextrin

5g Erythromycin and 62g hydroxide propyl beta-cyclodextrin (critical mole ratio of the two is 6) are dissolved in 200ml ethanol by heating. After active carbon is added, color and pyrogen is removed. The solution is heated for 15 minutes, and filtered. The filter liquor is added in rotation pot with volume of 1 litre, and kept at 70∼80°C. The liquor is decompressed, concentrated and converted to aqueous system, then continuously decompressed until the materiel is expanded and dried. yield: ≥98%.

### Example 4

### Process of preparing a complex of Azithromycin-hydroxide propyl beta-cyclodextrin

1g Azithromycin and 34g hydroxide propyl beta-cyclodextrin (critical mole ratio of the two is 16) are dissolved in 100ml ethanol by heating. After active carbon is added, color and pyrogen is removed. The solution is heated for 15 minutes, and filtered. The filter liquor is added in rotation pot with volume of 1 litre, and kept at 70∼80°C. The liquor is decompressed and concentrated before solvent is recycled and converted to aqueous system, then continuously decompressed until the materiel is expanded and dried for 2.5∼3h. The total time is about 3.5∼4h. yield: ≥98%. Solvent recovery rate: 85∼90%.

### Example 5

### Process of preparing a complex of Medicamycin-hydroxide propyl beta-cyclodextrin

3g Medicamycin and 53g hydroxide propyl beta-cyclodextrin (critical mole ratio of the two is 8) are dissolved in 100ml tetrahydrofuran by heating. After active carbon is added, color and pyrogen is removed. The solution is filtered. The filter liquor is added in rotation pot with volume of 1 litre, and kept at 70∼80°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 85∼90%.

### Example 6

### Process of preparing a complex of Roxithromycin-hydroxide propyl beta-cyclodextrin

2g Roxithromycin and 72g hydroxide propyl beta-cyclodextrin (critical mole ratio of the two is 18) are dissolved in 100ml ethanol by heating. After active carbon is added, color and pyrogen is removed. The solution is filtered. The filter liquor is added in rotation pot with volume of 1 litre, and kept at 80°C in 120r/min rotation speed. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 90%.

Preparing complexes of other macrolide antibiotics are similar to the above-mentioned preparations.

### Example 7

### Process of preparing a complex of Itraconazole-hydroxide propyl beta-cyclodextrin

1g Itraconazole and 40g hydroxide propyl beta-cyclodextrin (critical mole ratio of the two is 18) are dissolved in appropriate amount of ethanol by heating. After active carbon is added, color and pyrogen is removed. The solution is filtered. The filter liquor is added in rotation pot with volume of 1 litre, and kept at 65∼70 °C. The liquor is decompressed and concentrated before solvent is recycled and converted to aqueous system, then continuously decompressed until the materiel is expanded and dried for 2.5∼3h. The total time is about 3.5∼4h. yield: ≥98%. Solvent recovery rate: 90%.

### Example 8

### Process of preparing complex of Ketoconazole-hydroxide propyl beta-cyclodextrin

5g Ketoconazole and 45g hydroxide propyl beta-cyclodextrin (critical mole ratio of the two is 3) are dissolved in ethanol and tween 80 1.5g. After active carbon is added, color and pyrogen is removed. The solution is filtered. The filter liquor is added in rotation pot with volume of 1 litre, and kept at 65∼70°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 90%.

### Example 9

### Process of preparing a complex of Rotundine-hydroxide propyl beta-cyclodextrin

5g Rotundine and 23g hydroxide propyl beta-cyclodextrin (critical mole ratio of the two is 1) are dissolved in ethanol and the solution is filtered if necessary. The filter liquor is added in rotation pot with volume of 1 litre, and kept at 60∼65°C. The liquor is decompressed and concentrated before solvent is recycled and converted to aqueous system, then continuously kept and decompressed until the materiel is expanded and dried. yield: ≥98%. Solvent recovery rate: 85∼90%. Caution: operated in no light (direct light).

### Example 10

### Process of preparing a complex of Lappaconitine-hydroxide propyl beta-cyclodextrin

5g hydrobromic Lappaconitine and 28g hydroxide propyl beta-cyclodextrin (critical mole ratio of the two is 2) are dissolved in ethanol and the solution is filtered if necessary. The filter liquor is added in rotation pot with volume of 1 litre, and kept at 55∼65°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 90%.

### Example 11

### Process of preparing a complex of lorazepam-hydroxide propyl beta-cyclodextrin

3g lorazepam and 50g hydroxide propyl beta-cyclodextrin (critical mole ratio of the two is 3) are dissolved in ethanol and the solution is filtered if necessary. The filter liquor is added in rotation pot with volume of 1 litre, and kept at 70°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 90%.

### Example 12

### Process of preparing a complex of Zolpidem-hydroxide propyl beta-cyclodextrin

10g tartaric acid Zolpidem and 42g hydroxide propyl beta-cyclodextrin (critical mole ratio of the two is 2) are dissolved in ethanol and the solution is filtered if necessary. The filter liquor is added in rotation pot, and kept at 60∼70°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 85∼90%.

### Example 13

### Process of preparing a complex of Tamoxifen-hydroxide propyl beta-cyclodextrin

5g Tamoxifen and 32g hydroxide propyl beta-cyclodextrin (critical mole ratio of the two is 2) are dissolved in ethanol and the solution is filtered if necessary. The filter liquor is added in rotation pot with volume of 1 litre, and kept at 60∼70°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 90%.

### Example 14

### Process of preparing a complex of Etoposide-hydroxide propyl beta-cyclodextrin

2g Etoposide and 55g hydroxide propyl beta-cyclodextrin (critical mole ratio of the two is 10) are dissolved in ethyl acetate by heating. After active carbon is added, color and pyrogen is removed. The solution is kept for 15 minutes and filtered. The filter liquor is added in rotation pot with volume of 1 litre, and kept at 70∼80°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 90%.

### Example 15

### Process of preparing a complex of busulfan-hydroxide propyl beta-cyclodextrin

2g busulfan and 66g hydroxide propyl beta-cyclodextrin (critical mole ratio of the two is 5) are dissolved in appropriate amount of ethanol by heating. After active carbon is added, color and pyrogen is removed. The solution is kept for 15 minutes and filtered. The filter liquor is added in rotation pot with volume of 1 litre, and kept at 75∼80°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 85∼90%.

### Example 16

### Process of preparing a complex of Paclitexel-hydroxide propyl beta-cyclodextrin

0.5g Paclitexel and 90g hydroxide propyl beta-cyclodextrin (critical mole ratio of the two is 100) are dissolved in ethanol by heating. After active carbon is added, color and pyrogen is removed. The solution is filtered. The filter liquor is added in rotation pot with volume of 1 litre, and kept at 60∼70°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 85%.

### Example 17

### Process of preparing a complex of prednisone-hydroxide propyl beta-cyclodextrin

2g prednisone and 36g hydroxide propyl beta-cyclodextrin (critical mole ratio of the two is 4) are dissolved in tetrahydrofuran by heating. After active carbon is added, color and pyrogen is removed. The solution is filtered. The filter liquor is added in rotation pot with volume of 1 litre, and kept at 65∼75°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 85∼90%.

### Example 18

### Process of preparing a complex of Prasterone-hydroxide propyl beta-cyclodextrin

3g Prasterone and 54g hydroxide propyl beta-cyclodextrin (critical mole ratio of the two is 3) are dissolved in ethanol and the solution is filtered if necessary. The filter liquor is added in rotation pot with volume of 1 litre, and kept at 65∼75°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 85∼90%.

### Example 19

### Process of preparing a complex of spironolactone-hydroxide propyl beta-cyclodextrin

4g spironolactone and 48g hydroxide propyl beta-cyclodextrin (critical mole ratio of the two is 3) are dissolved in ethanol by heating. After active carbon is added, color and pyrogen is removed. The solution is filtered. The filter liquor is added in rotation pot with volume of 1 litre, and kept at 55∼65 °C. The liquor is decompressed and concentrated before solvent is recycled and converted to aqueous system, then continuously kept and decompressed until the materiel is expanded and dried for 2.5∼3h. The total time is about 3.5∼4h. yield: ≥98%.

Solvent recovery rate: 85%.

### Example 20

### Process of preparing a complex of Dioxopromethazine-hydroxide propyl beta-cyclodextrin

20g Dioxopromethazine hydrocloride and 40g hydroxide propyl beta-cyclodextrin (critical mass ratio of the two is 2) are dissolved in ethanol and the solution is filtered if necessary. The filter liquor is added in rotation pot with volume of 1 litre, and kept at 65∼75°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 90%.

### Example 21

### Process of preparing a complex of Bromohexamine-hydroxide propyl beta-cyclodextrin

3g Bromohexamine hydrocloride and 36g hydroxide propyl beta-cyclodextrin (critical mole ratio of the two is 3) are dissolved in ethanol and the solution is filtered if necessary. The filter liquor is added in rotation pot with volume of 1 litre, and kept at 65∼75°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 85∼90%.

### Example 22

### Process of preparing a complex of sulfamethoxazole-hydroxide propyl beta-cyclodextrin

4g sulfamethoxazole and 56g hydroxide propyl beta-cyclodextrin (critical mole ratio of the two is 2) are dissolved in ethanol and the solution is filtered if necessary. The filter liquor is added in rotation pot with volume of 1 litre, and kept at 65∼75°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 85%.

### Example 23

### Process of preparing a complex of TMP -hydroxide propyl beta-cyclodextrin

3g TMP and 50g hydroxide propyl beta-cyclodextrin (critical mole ratio of the two is 3) are dissolved in ethanol and the solution is filtered if necessary. The filter liquor is added in rotation pot with volume of 1 litre, and kept at 65∼75°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 85%.

### Example 24

### Process of preparing a complex of Ampicilline-hydroxide propyl beta-cyclodextrin

2g Ampicilline and 28g hydroxide propyl beta-cyclodextrin (critical mole ratio of the two is 3) are dissolved in ethanol and the solution is filtered if necessary. The filter liquor is added in rotation pot with volume of 1 litre, and kept at 60∼70°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 90%.

### Example 25

### Process of preparing a complex of Metronidazole-hydroxide propyl beta-cyclodextrin

4g Metronidazole and 36g hydroxide propyl beta-cyclodextrin (critical mass ratio of the two is 9) are dissolved in ethanol and the solution is filtered if necessary. The filter liquor is added in rotation pot with volume of 1 litre, and kept at 60∼70 °C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 85%.

### Example 26

### Process of preparing a complex of caffein-hydroxide propyl beta-cyclodextrin

8g caffein and 32g hydroxide propyl beta-cyclodextrin (critical mass ratio of the two is 4) are dissolved in ethanol and the solution is filtered if necessary. The filter liquor is added in rotation pot with volume of 1 litre, and kept at 60∼70°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 90%.

### Example 27

### Process of preparing a complex of paracetamol-hydroxide propyl beta-cyclodextrin

10g paracetamol and 30g hydroxide propyl beta-cyclodextrin (critical mass ratio of the two is 3) are dissolved in ethanol and the solution is filtered if necessary. The filter liquor is added in rotation pot with volume of 1 litre, and kept at 60∼70 °C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 90%.

### Example 28

### Process of preparing a complex of nimodipine-hydroxide propyl beta-cyclodextrin

0.5g nimodipine and 68g hydroxide propyl beta-cyclodextrin (critical mole ratio of the two is 35) are dissolved in ethanol by heating. After active carbon is added, color and pyrogen is removed. The solution is filtered. The filter liquor is added in rotation pot with volume of 1 litre, and kept at 70∼80°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 85∼90%.

### Example 29

### Process of preparing a complex of cinnarizine-hydroxide propyl beta-cyclodextrin

4g cinnarizine, 53g hydroxide propyl beta-cyclodextrin (critical mole ratio of the two is 3), 2.5g citric acid and slight amount of tween80 are dissolved in tetrahydrofuran by heating. After active carbon is added, color and pyrogen is removed. The solution is filtered. The filter liquor is added in rotation pot with volume of 1 litre, and kept at 60∼70°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 90%.

### Example 30

### Process of complex of Sildenafil-hydroxide propyl beta-cyclodextrin

3g citric acid Sildenafil citrate and 15g hydroxide propyl beta-cyclodextrin (critical mole ratio of the two is 2) are dissolved in ethanol and the solution is filtered if necessary. The filter liquor is added in rotation pot with volume of 1 litre, and kept at 65∼75°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 90%.

### Example 31

### Process of complex of famotidine-hydroxide propyl beta-cyclodextrin

5g famotidine, 45g hydroxide propyl beta-cyclodextrin (critical mass ratio of the two is 9) and 2.5g citric acid is dissolved in ethanol and the solution is filtered if necessary. The filter liquor is added in rotation pot, and kept at 60∼70°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 85%.

### Example 32

### Process of preparing a complex of Ciclosporin A - hydroxide propyl beta-cyclodextrin

0.5g Ciclosporin A and 43g hydroxide propyl beta-cyclodextrin (critical mole ratio of the two is 66) are dissolved in mixture of ethanol and tetrahydrofuran, and the solution is filtered if necessary. The filter liquor is added in rotation pot with volume of 1 litre, and kept at 60∼70°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 85∼90%.

### Example 33

### Process of preparing a complex of Lovastatin-hydroxide propyl beta-cyclodextrin

0.5g Lovastatin and 52g hydroxide propyl beta-cyclodextrin (critical mole ratio of the two is 26) are dissolved in dimethyl formamide and the solution is filtered if necessary. The filter liquor is added in rotation pot with volume of 1 litre, and kept at 60∼70°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 85∼90%.

### Example 34

### Process of preparing a complex of simvastatin-hydroxide propyl beta-cyclodextrin

0.5g simvastatin and 56g hydroxide propyl beta-cyclodextrin (critical mole ratio of the two is 29) are dissolved in ethanol and the solution is filtered if necessary. The filter liquor is added in rotation pot with volume of 1 litre, and kept at 60∼70 °C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 85∼90%.

### Example 35

### Process of preparing a complex of menthol-hydroxide propyl beta-cyclodextrin

2.5g menthol and 50g hydroxide propyl beta-cyclodextrin (critical mole ratio of the two is 2) are dissolved in mixture of acetone and ethanol. The solution is added in rotation pot with volume of 1 litre, and kept at 50∼65°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 90%.

### Example 36

### Process of preparing a complex of borneol-hydroxide propyl beta-cyclodextrin

6g borneol and 36g hydroxide propyl beta-cyclodextrin (critical mass ratio of the two is 6) are dissolved in ethanol. The solution is added in rotation pot with volume of 1 litre, and kept at 55∼65°C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 85%.

### Example 37

### Process of preparing a complex of chlorbutanol-hydroxide propyl beta-cyclodextrin

6g chlorbutanol and 36g hydroxide propyl beta-cyclodextrin (critical mass ratio of the two is 6) are dissolved in methanol and the solution is filtered if necessary. The filter liquor is added in rotation pot with volume of 1 litre, and kept at 55∼65 °C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 85%.

### Example 38

### Process of preparing a complex of ligustrazine-hydroxide propyl beta-cyclodextrin

3g ligustrazine and 36g hydroxide propyl beta-cyclodextrin (critical mass ratio of the two is 12) are dissolved in mixture of ethanol and methanol by heating. After active carbon is added, color and pyrogen is removed. The solution is filtered. The filter liquor is added in rotation pot with volume of 1 litre, and kept at 55∼65 °C. The liquor is decompressed and concentrated before solvent is recycled and converted to aqueous system, then continuously kept and decompressed until the materiel is expanded and dried for 2.5∼3h. The total time is about 3.5∼4h. yield: ≥98%. Solvent recovery rate: 85%.

Complexes of the more than 60 medicines and compounds generated as β-CD replaced by hydroxide propyl, ethoxyl, sulphur alkyl, ethyl ether, methyl and ethyl, are extremely freely water-soluble or freely water-soluble as dissolved in water, furthermore, dissolving speed is fast. The complex is in possession of infinite dilution stability when its solution is diluted with water again.

The results of present experiment indicate: complexes of the medicine and hydroxide propyl beta-cyclodextrin, or many beta-cyclodextrin derivatives, do not impact on medicine efficacy, but often increase efficacy. They do not increase toxicity and side effect, but often decrease them; as dose types and routes of administration are changed, effects of increasing medicine efficacy are more obvious. For instance, experiment results of medicine efficacy on killing plasmodium with complex of Artemisinin and hydroxide propyl beta-cyclodextrin in mice infected plasmodium, Artemisinin is illustrated in table 2, of which, Artesunate, and their complex of hydroxide propyl beta-cyclodextrin is indicated as Artemisinin-hydroxide propyl beta-cyclodextrin and Artesunate-hydroxide propyl beta-cyclodextrin.

**Table 2:**

| compare efficacy of complexes of two kinds of Artemisinin classes and hydroxide propyl beta-cyclodextrin with efficacy of original medicine | | | | |
|---|---|---|---|---|
| name | route of administration | efficacy (killing) | | ED₉₀comparison p.o./iv |
| | | ED₅₀ | ED₉₀ | |
| Artemisinin | po. | 19 | 49 | 2.13 |
| Artemisinin-hydroxide propyl beta-cyclodextrin | iv. | 5 | 23 | 1 |
| Artesunate | po. | 9.96 | 25.13 | 1.88 |
| Artesunate-hydroxide propyl beta-cyclodextrin | iv. | 4.32 | 13.37 | 1 |

From above-mentioned, it indicates: medicine efficacy is increased respectively by 2.13 and 1.88 times, while side effects are not increased remarkably.

Another example: hydrochloric Sibutramine-HP-β-CD complex (water-soluble) is prepared to promptly soluble oral tablet. Compared with oral capsule in animal experiment(dog), bioavailability of promptly soluble oral tablet is higher than oral capsule by 1 time or more. The oral soluble tablet of Tartaric Zolpidem takes effect faster than common tablet, while bioavailability is improved by 1 time.

### Industrial Application

The methods of present invention are advantageous in that: 1. The technique is more simple than existing techniques, and the manufacturing cycle is short, generally only 3∼4 hours; 2. Acid, alkali surface active agent and cosolvent are unnecessary to be added in, which results in that medicine efficacy is enhanced, and side effects are decreased; 3. After an organic medicine is dissolved in water or organic solvent, and converted to an aqueous solution, a complex is generated by molecule assembling. Then a loose multihole solid is prepared, which is extremely freely water-soluble or freely water-soluble, with fast dissolving speed and infinite dilution stability; 4. The critical value between organic medicine and beta-cyclodextrin derivatives is determined by unit test and preparing saturated solution, so based on the critical value complex structure prepared becomes more stable, etc.

On the basis of the methods of present invention, all of water-insoluble or sparingly-soluble organic medicines or other organic compounds are prepared to water-soluble and sterile complex. Thus, appreciably convenient means are provided for industrial uses of such organic medicine preparation and of other organic compounds.

## Claims

1. A preparation method of complex of organic medicine and beta-cyclodextrin derivative comprising the following steps of:
a. successively or simultaneously dissolving organic medicine and beta-cyclodextrin derivative in organic solvent at certain mole ratio or mass ratio in the presence of suitable amount of water;
b. removing the organic solvent from the solution of the step a to obtain aqueous system of the organic medicine and beta-cyclodextrin derivative; and
c. removing water from the aqueous system to obtain the complex of the organic medicine and the beta-cyclodextrin derivative.

2. The method according to claim 1, wherein the organic solvent is hydrophilic solvent.

3. The method according to claim 2, wherein the organic hydrophilic solvent is selected from ester solvent of lower fatty acid, hydrocarbon solvent, halohydrocarbon solvent, furan solvent, amide solvent, lower fatty alcohol, nitrile solvent, ketone solvent, and their mixtures.

4. The method according to claim 3, wherein the organic hydrophilic solvent is selected from methyl acetate, ethyl acetate, butyl acetate, benzinum, cyclohexane, methylene chloride, chloroform, water, tetrahydrofuran, dimethylacetamide, dimethyl formamide, methanol, ethanol, propanol, butanol, acetonitrile, acetone, and their mixtures.

5. The method according to claim 1, wherein the dissolving in the step a is carried out at 30∼100°C.

6. The method according to claim 5, wherein the dissolving in the step a is carried out at 60∼75°C.

7. The method according to claim 1, wherein the mole ratio or mass ratio of the organic medicine and the beta-cyclodextrin derivative is the critical mole ratio or the critical mass ratio, or less than the critical mole ratio or critical mass ratio, or more than the critical mole ratio or the critical mass ratio.

8. The method according to claim 1, wherein the critical mole ratio and critical mass ratio of the organic medicine and beta-cyclodextrin derivative is determined by unit test or preparing saturated solution.

9. The method according to claim 1, wherein the beta-cyclodextrin derivative is selected from hydroxy propyl beta-cyclodextrin, ethoxyl beta-cyclodextrin, sulfoalkyl beta-cyclodextrin, ether beta-cyclodextrin, methyl beta-cyclodextrin, and ethyl beta-cyclodextrin.

10. The method according to claim 9, wherein the beta-cyclodextrin derivative is hydroxide propyl beta-cyclodextrin.

11. The method according to claim 1, wherein the method also comprises the step of sterile filtration of the solution obtained in the step a.

12. The method according to claim 1, wherein the organic medicine is water-insoluble or sparingly-soluble organic medicine.

13. The method according to claim 12, wherein the organic medicine is selected from the following medicines or their mixtures:
A. antimalarial: sesquiterpene lactone artemisinin including artemisinin, dihydroartemisinin, artemether, artesunate and artemoter;
B. macrolide antibiotics comprising erythromycin, medicamycin, roxithromycin, azithromycin and clarithromycin;
C. azole antifungal comprising Itraconazole, ketoconazole, miconazole and clotrimazole;
D. alkaloid analgesic comprising lappaconitine and rotundine;
E. anxiolytic and sleping potion comprising diazepam, lorazepam, estazolam, oryzanol and zolpidem;
F. antineoplastics comprising tamoxifen, paclitexel, busulfan and etoposide.
G. steroid comprising prednisone, testosterone,Prasterone and spironolactone;
H. immunosuppressant comprising ciclosporin;
I. anlibechic and expectorant comprising dioxopromethazin and bromohexamine;
J. Sulfa and synergist comprising sulfamethoxazole, sulfafurazole, sulfadiazine and TMP;
K. antibacterial agent comprising ampicilline, ciprofloxacin, furazolidone, norfloxacin, metromdazole and tinidazole;
L. antipyretic, anti-inflammatory agent, analgesic comprising paracetamol, proxicam and ibuprofen;
M. medicines for circulatory system comprising nimodipine, nitrendipine, cinnarizine, molsidomine, lovastatin and simvastatin; and
N. other medicines and volatile material comprising famotidine, sibutramin, sildenafil, gliclazid, difenidol, caffeine, ketotifen, lipoicacid, thioctamide, menthol, borneol, camphor, ligustrazine, chlorbutanol and vanillin.

14. The method according to claim 13, wherein the complex is heated and expanded in vacuum, dried, and made into multihole sterile granula or powder.

15. The method according to claim 13, wherein the complex is directly sub-packed to prepare powder and injection or freeze drying powder, mass or solution.

16. The method according to claim 13, wherein the complex is used together with adjuvant approved in pharmacy to prepare tablet, granula, capsule, pill, chewing agent, suppository, adhibition agent.

17. The method according to claim 13, wherein the complex is made into aerosol inhalation.

18. The method according to claim 13, wherein the complex is made into pharmaceutical solution for eye drip, nasal drip, gargle, inhalator, rectum, washing.

19. The method according to claim 13, wherein the complex is made into the medicine, hormone and nutrient of prevention and treatment animals, poultries, agronomic crops and plants except human.

20. The method according to claim 13, wherein the complex is used to prepare enzyme preparation and catalyst in chemical industry.

21. A complex of the organic medicine and beta-cyclodextrin derivative prepared with the method according to any one of claims 1-20.
